# EUROPEAN PATENT SPECIFICATION

(11) **EP 0 367 422 B1**
(45) Date of publication and mention of the grant of the patent: **21.12.1994**
(21) Application number: 89310334.1
(22) Date of filing: 11.10.1989
(51) Int. Cl.: A61M 5/00

(54) **Closure for container**
Behälterverschluss
Fermeture pour récipient

(30) Priority: 11.10.1988 GB 8823834
(43) Date of publication of application: 09.05.1990
(73) Proprietor: Rexam Plastic Packaging Limited, Hereford HR2 6LD (GB)
(72) Inventor: Rogers, Paul Terence, Lugwardine Hereford HR1 4AA (GB)
(74) Representative: Stuart, Ian Alexander

(56) References cited:
- WO-A-88/00067
- DE-U- 8 808 135
- US-A- 4 375 849
- US-A- 4 466 538
- US-A- 4 657 139

## Description

The present invention relates to a closure for a container, particularly a disposable container. It further relates to a container including such a closure.

It is commonplace in hospitals and other places where syringes are used to provide special containers for the disposal of syringe needles and other contaminated "sharps". Once the "sharps container" is full, it should be closed and taken away for disposal, preferably by incineration. It is desirable that once the container is finally closed, it should not be opened again.

WO 88/00067 discloses a container for used syringe needles closed at the top by a plate with an aperture. An apertured cap is rotatably mounted over the plate so that its aperture can be moved into and out of register with the plate's aperture. This is for gripping a needle projecting through the apertures. It is stated that a further position or positions may be provided whereby the cap can be rotated to a "locked" position with respect to the plate when the container is full. However, there is no disclosure of how this could be effected. Furthermore, there is no disclosure of means for restraining removal of the plate and cap.

US-A-4,657,139 discloses a syringe container having a lid bearing a rotatable closure panel with a downwardly projecting boss. To lock the panel closed, it is rotated until the boss, which is normally resiliently urged against the lid, drops into an aperture in the lid. Other closures for syringe containers are disclosed in US-A-4,375,849 , US-A-4,466,538 and DE-U-8,808,135.

The present invention concerns a closure assembly for a container, comprising a wall member for connection to a mouth of a container, the wall member having an access aperture; and an obstructor member slidably mounted on the wall member so as to be slidable between an obstructing configuration in which it obstructs the access aperture, and an open configuration in which any obstruction by it of the access aperture is less than in the obstructing configuration; and wherein the closure assembly includes a locking piece associated with a first one of the obstructor member and the wall member, and a detent formation associated with the second one of said members, the arrangement being such that the locking member is engageable behind the detent formation thereby to lock the obstructor member in its obstructing configuration and restrain its return to its open configuration; characterised in that the locking member comprises pin means slidably mounted to said first member so as to be selectively displaceable from a first position to a second position in which it projects to a greater extent towards the other member; said detent formation being arranged to be engageable or unengageable by the pin means depending on the extent of projection of the pin means. Thus the access aperture may be defined in a surface over which the closure member is adapted to slide. The closure member may carry a locking pin adapted to be moved irreversibly to engage a detent formation in the surface, so as to prevent the sliding of the closure member away from the main access opening. (Alternatively, the pin could be borne by the surface member, and engage a detent formation of the closure member.)

In another aspect the invention provides a container having such a closure assembly, connected or connectable so as to be difficult or impossible to detach from the body of the container.

Embodiments of the invention will now be described in greater detail with reference to the accompanying drawings in which:
Fig. 1 is a top plan view of a container lid for use in an embodiment of the invention;
Fig. 2 is a vertical section elevation of a container including the lid shown in Fig. 1;
Fig. 3 is a plan view of a closure member;
Fig. 4 is a section on V-V in Fig. 3;
Fig. 5 is an enlarged detail of Fig. 4, and also shows a locking pin;
Fig. 6 is a schematic top plan view of a modified embodiment of a container lid;
Fig. 7 is a partly sectional view in elevation of the lid shown in Fig. 6;
Fig. 8 is an axial section through a modified form of locking pin, with a detail of a modified form of lid;
Fig. 9 is a vertical section through a second embodiment of container lid; and
Fig. 10 is a detail of a plan view of a container lid showing variant needle detachment formations.

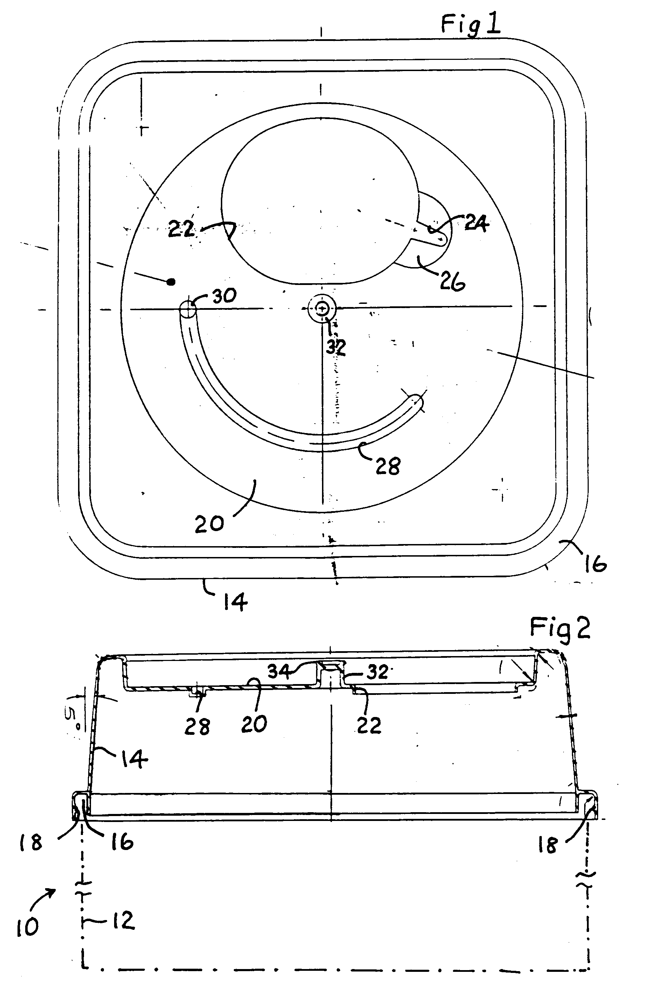

Referring first to Figs. 1 to 5, a container 10 (whose body 12 is shown schematically in Fig. 2) has an upper wall member or lid 14 whose mouth has a downwardly open channel 16 with an inwardly directed bead 18 for snap-engaging a complementary formation at the top of the body 12 (not shown). The upper face of the lid 14 has a recessed circular portion 20 penetrated by an access aperture 22. The aperture 22 is approximately elliptical. It may include a narrow extension 24 projecting into a region 26 where the lid 14 is of reduced thickness. This inlet 24 provides a means for detaching needles from hypodermic syringes.

The recess 20 has an arcuate depression 28, which is of uniform depth except for a deep aperture 30 at the end nearest the opening 22. In the centre of the recess 20, there is an upstanding pillar 32 which has a projecting bead 34 adjacent its top.

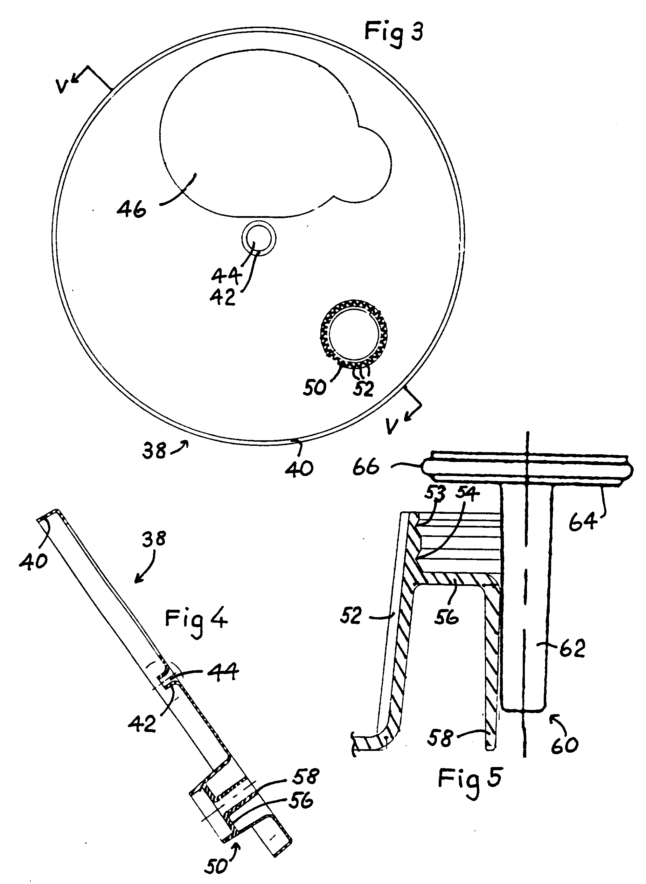

Figs. 3 and 4 show an obstructor member 38 in the form of a disc dimensioned to fit within the recess 20. The disc has a peripheral upturned lip 40 and a central upturned wall portion 42 defining a bushing having a central aperture 44. This is dimensioned so that the disc 38 can fit into the recess 20, with the pillar 32 extending through the aperture 44. The disc is a push-fit, such that the bead 34 snap-engages above the bushing 42, to retain the disc rotatably on the lid.

The disc has a large aperture 46 similar to the access opening 22 and thinned area 26 of the lid, with which it can be brought into register by rotation. The disc has a handgrip 50 defined by a frustoconical wall with external serrations 52. As can be seen from Figs. 4 and 5, the wall 50 has an upper portion whose internal surface is formed with a vertically spaced pair of annular recesses 53,54. Beneath them there is a radially extending annular wall 56, leading to a central tubular portion 58. A locking pin 60 has a shank 62 dimensioned to be slidable within the tube 58 so that it can project beneath the tube 58 by variable amounts. The pin 60 has a head 64 including a radially outer curved bead portion 66. This is shaped and dimensioned so that it can locate in the upper annular groove 53. In this configuration, the shank 62 projects a short way beneath the tubular portion 58 and the rest of the disc 38. The projecting end portion extends into the arcuate recess 28, and thus guides and delimits the rotation of the disc. When the disc is rotated so that its main aperture 46 is in register with the access opening 22, the pin 60 is at the right-hand end of the arcuate recess 28, as seen in Fig. 1. To close the access opening 22, the disc 38 is rotated clockwise as far as it can go. Thus the pin moves to the other end of the arcuate recess 28, in the region of the deep portion 30. If it is desired to lock the disc in the closing configuration, application of downward pressure to the locking pin enables it to travel downwardly, the bead 66 jumping from the upper annular recess 53 to the lower recess 54. The shank 62 then extends into the deep recess 30, and prevents rotation of the disc.

The container is suitably made of a plastics material, e.g. polypropylene. A full container may be incinerated intact, with its contents.

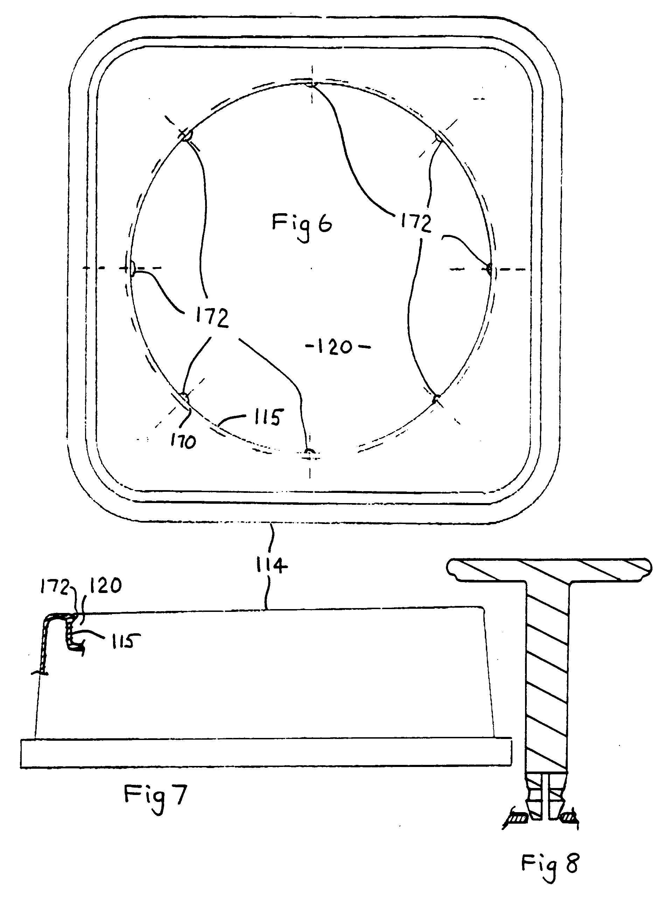

The obstructor disc 38 is secured to the lid 14 only by the engagement of the bead 34 of the central pillar 32 of the lid above the bushing 42. Figs. 6 and 7 show how the lid 114 may be modified to enhance the security of the connection. The peripheral wall 115 delimiting the circular recess 120 may have an annular bead 170 behind which the disc 38 snap-engages. However, unless this is very slight, it makes it very difficult to produce lids by injection moulding and free them from the mould. Therefore, a plurality of radially projecting pips 172 are provided as well as (or instead of) the bead 170, for the disc 38 to engage behind. This makes the disc 38 very difficult to separate from the lid, particularly when access from behind is impossible, as it is when the lid is locked on a container.

Fig. 8 shows another way of strengthening the connection of the disc 38 and the lid. This operates only when the locking pin is depressed to lock the disc in the closing configuration. It involves modifications to both the locking pin and the lid. The modified pin 160 has a shank with a tapered end portion 174 leading to an annular groove 176. To increase compressibility of the end portion, there may be an axial slot 178. In the lid 114, the locking end of the arcuate depression 28 provides a simple hole 180 through the thickness of the lid, rather than a blind aperture 30. The diameter of the hole is between the minimum and maximum diameters of the tapered end portion 174 of the pin. Thus when the pin is depressed for locking, its end portion is forced through the hole 180 (with compression or deformation), and the pin becomes locked to the lid because the lid margin around the hole engages in the annular groove 176. The pin thus clamps the disc to the lid. (There may be formations 52-58 on the disc substantially as shown in Fig. 5, though the snap-engagement of the pin's head 166 in a lower annular recess 54 may not now be necessary.)

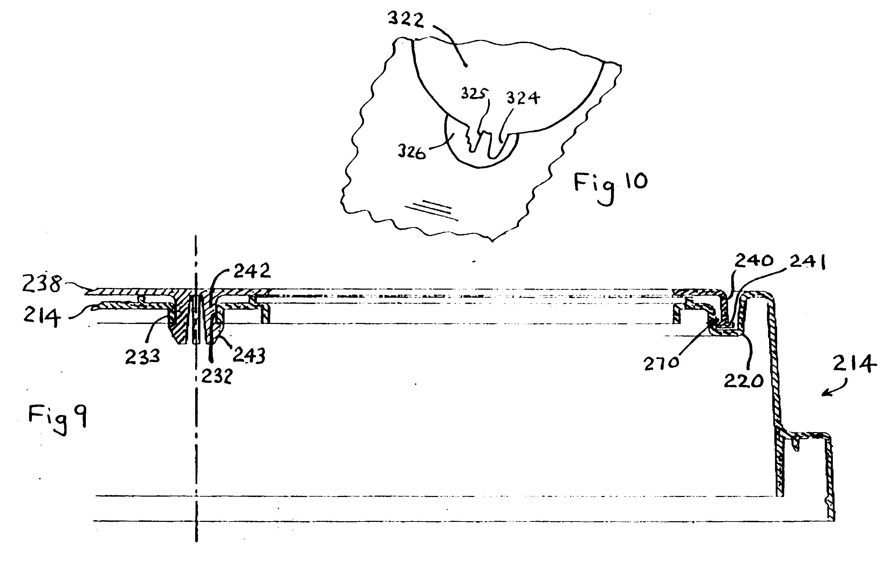

Fig. 9 shows another form of container lid 214 and obstructor member 238. The latter again has the form of a disc with a peripheral lip 240, but this is down-turned and ends in a radially exrending flange 241. The lid 214 has a circumferential channel 220 in which the lip 240 engages. The radially inner wall of the channel 220 has a circumferentially extending projection 270 which serves as a clip beneath which the flange 241 engages. The disc 238 has a central downwardly-extending backing 242 with an enlarged head 243. The lid 214 has a central opening 232 delimited by a downturned lip 233 dimensioned so that the bushing 242 of the disc 238 can be pushed into the aperture until the head 243 is forced past the lip 233, beneath which it becomes trapped. This engagement and that of the lip 240 in the channel 220 allow the disc to rotate but resist its withdrawal.

Fig. 10 shows a portion of a lid's access aperture 322. Like the aperture 22 shown in Fig. 1, its margin includes a thinned region 326 into which a narrow inlet 324 opens, this inlet being slightly tapered and intended for use in disengaging a needle from a syringe. This variant includes an alternative inlet 325 whose width is reduced stepwise. This is to allow a syringe to be engaged therewith by rotation, whereafter it can be pulled free of its needle.

## Claims

1. A closure assembly for a container, comprising a wall member (14;114) for connection to a mouth of a container (12), the wall member (14;114) having an access aperture (22); and an obstructor member (38) slidably mounted on the wall member (14;114) so as to be slidable between an obstructing configuration in which it obstructs the access aperture (20), and an open configuration in which any obstruction by it of the access aperture is less than in the obstructing configuration; and wherein the closure assembly includes a locking piece (60;160) associated with a first one of the obstructor member and the wall member, and a detent formation (30;180) associated with the second one of said members, the arrangement being such that the locking member is engageable behind the detent formation thereby to lock the obstructor member (38) in its obstructing configuration and restrain its return to its open configuration; characterised in that
the locking member (60;160) comprises pin means slidably mounted to said first member so as to be selectively displaceable from a first position to a second position in which it projects to a greater extent towards the other member; said detent formation (30; 180) being arranged to be engageable or unengageable by the pin means (60;160) depending on the extent of projection of the pin means.

2. A closure assembly according to claim 1 wherein said other member (14;114) has an elongate guide formation (28) arranged to be contacted by said pin means (60;160) when it is in its first position in which it projects to a lesser extent, the guide formation (28) acting to guide the relative sliding of the obstructor member (38) and the wall member (14;114).

3. A closure assembly according to any preceding claim wherein displacement of the locking piece (160) to lock the obstructor member (38) in its locking configuration serves also to lock the obstructor member (38) to the wall member (114).

4. A closure assembly according to claim 3 wherein the pin means (160) has a groove (176) at its projecting end and said other member (114) has a hole (180) disposed and dimensioned so that displacement of the pin means (16) to effect locking causes its end portion to be forced through the hole (180) until the margin around the hole (180) engages in the groove (176), thereby locking the obstructor member (38) to the wall member (114)

5. A closure assembly according to any preceding claim having a tubular guide (58) for the displacement of the locking piece (60;160), the tubular guide (58) and locking piece (60;160) having mutual engagement formations (53,54,66) for engaging the locking piece (60;160) in its locking configuration.

6. A closure assembly according to any of claims 1-4 wherein the pin means has a shank (62) and an enlarged head (64,66) and the first member (38) has a tubular portion (58) within which the shank is slidable and an annular recess (54) dimensioned to receive the head (64,66) of the pin and disposed so that when the pin is displaced to its second, locking position, the head becomes engaged in said recess (54).

7. A closure assembly according to any preceding claim wherein the obstructor member (38) is a disc rotatably mounted on the wall member (60;160); the wall member has an arcuate recess (28) with a step formation (30) at one end region; and the pin means (60;160) has an end portion which travels in the arcuate recess (28) to delimit the relative displacement of the obstructor member and the wall member, the blocking configuration occurring when the pin means is at said end region; whereupon the pin means is displaceable so that abutment with the step formation restrains displacement from the blocking to the open configuration.

8. A closure assembly according to claim 7 wherein the wall member (114) is penetrated by an aperture (180) in said one end region of the recess; displacement of the pin means causing it to project into the aperture (180), whose edge constitutes said step formation.

9. A closure assembly according to claim 8 wherein the end region of the pin means (160) has a relatively large-diameter portion that is forced through the aperture (180) by said displacement and then engages beneath the wall member (160) to restrain withdrawal of the pin means (160) and thereby lock the obstructor member (38) to the wall member (114).

10. A container (12) having a mouth and a closure assembly for closing the mouth, said assembly, being according to any preceding claim.

## Patentansprüche

1. Verschlußanordnung für einen Behälter, umfassend ein Wandelement (14; 114) zur Verbindung mit einer Öffnung eines Behälters (12), wobei das Wandelement (14; 114) eine Zugangsöffnung (22) aufweist; und ein Verschlußelement (38), das gleitend am Wandelement (14; 114) angebracht ist, um zwischen einer Geschlossen-Stellung, in der es die Zugangsöffnung (20) verschließt und einer Offen-Stellung gleiten zu können, in der die Verschließwirkung auf die Zugangsöffnung geringer als in der Geschlossen-Stellung ist; und worin die Verschlußanordnung ein Verriegelungsstück (60; 160), das mit einem ersten des Verschlußelements und des Wandelements assoziiert ist, und eine Arretiereinrichtung (30; 180) enthält, die mit dem zweiten der genannten Elemente assoziiert ist, wobei die Anordnung solcherart ist, daß das Verriegelungselement hinter der Arretiereinrichtung in Eingriff bringbar ist, um dadurch das Verschlußelement (38) in seiner Geschlossen-Stellung zu verriegeln und seine Rückkehr zu seiner Offen-Stellung einzuschränken; dadurch gekennzeichnet, daß:
das Verriegelungselement (60; 160) einen Stift umfaßt, der gleitend am genannten ersten Element montiert ist, um selektiv von einer ersten Position in eine zweite Position verschiebbar zu sein, in der er in größerem Ausmaß hin zum anderen Element herausragt; wobei die genannte Arretiereinrichtung (30; 180) angeordnet ist, um durch den Stift (60; 160) je nach Ausmaß des Herausragens des Stifts in Eingriff oder außer Eingriff bringbar zu sein.

2. Verschlußanordnung nach Anspruch 1, worin das genannte andere Element (14; 114) eine längliche Führungseinrichtung (28) aufweist, die angeordnet ist, um mit dem genannten Stift (60; 160) in Kontakt zu stehen, wenn er sich in seiner ersten Position befindet, in der er zu einem geringeren Ausmaß herausragt, wobei die Führungseinrichtung (28) dazu dient, das relative Gleiten des Verschlußelements (38) und des Wandelements (14; 114) zu führen.

3. Verschlußanordnung nach einem der vorhergehenden Ansprüche, worin die Verschiebung des Verriegelungsstücks (160), um das Verschlußelement (38) in seiner Verriegelungsstellung zu verriegeln, auch dazu dient, das Verschlußelement (38) mit dem Wandelement (114) zu verriegeln.

4. Verschlußanordnung nach Anspruch 3, worin der Stift (160) an seinem herausragenden Ende eine Nut (176) aufweist und das genannte andere Element (114) ein Loch (180) aufweist, das solcherart angeordnet und dimensioniert ist, daß die Verschiebung des Stifts (16) zur Verriegelung zur Folge hat, daß sein Endabschnitt durch das Loch (180) gezwängt wird, bis der Rand um das Loch (180) in die Nut (176) eingreift, wodurch das Verschlußelement (38) mit dem Wandelement (114) verriegelt wird.

5. Verschlußanordnung nach einem der vorhergehenden Ansprüche, die eine rohrförmige Führung (58) zur Verschiebung des Verriegelungsstückes (60; 160) aufweist, wobei die rohrförmige Führung (58) und das Verriegelungsstück (60; 160) gegenseitige Eingriffseinrichtungen (53, 54, 66) zum Eingreifen in das Verriegelungsstück (60; 160) in seiner Verschlußkonfiguration aufweisen.

6. Verschlußvorrichtung nach einem der Ansprüche 1-4, worin der Stift einen Schaft (62) und einen vergrößerten Kopf (64, 66) aufweist und das erste Element (38) einen röhrenförmigen Abschnitt (58), in dem der Schaft gleiten kann und eine ringförmige Ausnehmung (54) aufweist, die so dimensioniert ist, um den Kopf (64, 66) des Stifts aufzunehmen und so angeordnet ist, daß beim Verschieben des Stifts zu seiner zweiten Position, der Verriegelungsposition, der Kopf in die genannte Ausnehmung (54) eingreift.

7. Verschlußanordnung nach einem der vorhergehenden Ansprüche, worin das Verschlußelement (38) eine drehbar auf dem Wandelement (60; 160) montierte Scheibe ist; das Wandelement eine bogenförmige Ausnehmung (28) mit einer Stufenausbildung (30) an einem Endbereich aufweist; und worin der Stift (60; 160) einen Endabschnitt aufweist, der sich in der bogenförmigen Ausnehmung (28) bewegt, um die relative Verschiebung des Verschlußelements und des Wandelements zu berschränken, wobei die Verschlußstellung eintritt, wenn sich der Stift im genannten Endbereich befindet; wodurch der Stift verschiebbar ist, sodaß ein Aneinanderstoßen mit der Stufenausbildung die Verschiebung von der verriegelten zur Offen-Stellung einschränkt.

8. Verschlußanordnung nach Anspruch 7, worin das Wandelement (114) durch eine Öffnung (180) im genannten einen Endbereich der Ausnehmung durchbrochen ist; wobei die Verschiebung des Stifts bewirkt, daß er in die Öffnung (180) ragt, deren Rand die genannte Stufenausbildung darstellt.

9. Verschlußanordnung nach Anspruch 8, worin der Endbereich des Stifts (160) einen Abschnitt mit relativ großem Durchmesser aufweist, der durch die genannte Verschiebung durch die Öffnung (180) gezwängt wird und dann unterhalb des Wandelements (160) in Eingriff tritt, um das Zurückziehen des Stifts (160) einzuschränken und um dadurch das Verschlußelement mit dem Wandelement (114) zu verriegeln.

10. Behälter (12) mit einer Öffnung und einer Verschlußanordnung zum Verschließen der Öffnung, wobei die genannte Anordnung einem der vorhergehenden Ansprüche entspricht.

## Revendications

1. Fermeture pour un récipient, comprenant un élément de paroi (14;114) pour la connexion à une embouchure d'un récipient (12), l'élément de paroi (14;114) possédant une ouverture d'accès (22); et un élément formant bouchon (38) monté de façon coulissante sur l'élément de paroi (14;114) de façon à coulisser entre une configuration d'obstruction dans laquelle il bouche l'ouverture d'accès (20) et une configuration ouverte dans laquelle toute obstruction par celui-ci de l'ouverture d'accès est plus petite que dans la configuration d'obstruction; et dans laquelle la fermeture comprend une pièce de verrouillage (60;160) associée à un premier des éléments d'obstruction et de paroi, et une formation de détente (30;180) associée au deuxième desdits éléments, l'agencement étant tel que l'élément de verrouillage puisse être mis en prise derrière la formation de détente en verrouillant ainsi l'élément formant bouchon (38) dans sa configuration d'obstruction et en empêchant son retour à sa configuration ouverte; caractérisée en ce que l'élément de verrouillage (60;160) comprend un moyen de broche monté de façon coulissante sur ledit premier élément de façon à être déplaçable sélectivement d'une première position vers une deuxième position dans laquelle il fait saillie suivant une plus grande étendue vers l'autre élément; ladite formation de détente (30;180) étant agencée pour pouvoir être mise en prise ou pour ne pas pouvoir être mise en prise avec le moyen de broche (60;160) en fonction de l'étendue de projection de l'élément de broche.

2. Fermeture selon la revendication 1, dans laquelle ledit autre élément (14;114) présente une formation de guidage allongée (28) agencée pour être contactée par ledit moyen de broche (60;160) lorsqu'il se trouve dans sa première position dans laquelle il fait saillie sur une étendue plus petite, la formation de guidage (28) agissant pour guider le coulissement relatif de l'élément formant bouchon (38) et de l'élément de paroi (14;114).

3. Fermeture selon l'une des revendications précédentes, dans laquelle le déplacement de la pièce de verrouillage (160) pour verrouiler l'élément formant bouchon (38) dans sa configuration de blocage sert également à verrouiller l'élément formant bouchon (38) à l'élément de paroi (114).

4. Fermeture selon la revendication 3, dans laquelle le moyen de broche (160) possède une rainure (176) à son extrémité en saillie, et ledit autre élément (114) présente un trou (180) disposé et dimensionné de façon que le déplacement du moyen de broche (16) pour provoquer le verrouillage amène sa partie d'extrémité à être forcée à travers le trou (180) jusqu'à ce que le bord autour du trou (180) s'engage dans la rainure (176) en verrouillant ainsi l'élément formant bouchon (38) à l'élément de paroi (114).

5. Fermeture selon l'une des revendications précédentes, présentant un guidage tubulaire (58) pour le déplacement de la pièce de verrouillage (60;160), le guidage tubulaire (58) et la pièce de verrouillage (60;160) présentant des formations d'engagement mutuelles (53,54,66) pour la mise en prise de la pièce de verrouillage (60;160) dans sa configuration de verrouillage.

6. Fermeture selon l'une des revendications 1 à 4, dans laquelle le moyen de broche présente une tige (62) et une tête élargie (64,66), et le premier élément (38) possède une partie tubulaire (58) à l'intérieur de laquelle la tige peut coulisser et un évidement annulaire (54) dimensionné de façon à recevoir la tête (64,66) de la broche et disposée de façon que lorsque la broche est déplacée à sa deuxième position de verrouillage, la tête s'engage dans ledit évidement (54).

7. Fermeture selon l'une des revendications précédentes, dans laquelle le moyen formant bouchon (38) est un disque monté de façon rotative sur l'élément de paroi (60;160); l'élément de paroi possède un évidement arqué (28) avec une formation étagée (30) à une région d'extrémité; et le moyen de broche (60;160) présente une partie d'extrémité qui se déplace dans l'évidement arqué (28) pour délimiter le déplacement relatif de l'élément formant bouchon et de l'élément de paroi, la configuration de blocage se produisant lorsque le moyen de broche se trouve à ladite région d'extrémité; par quoi le moyen de broche est déplaçable de telle sorte que la butée avec ladite formation étagée restreint le déplacement de la configuration de blocage à la configuration ouverte.

8. Fermeture selon la revendication 7, dans laquelle l'élément de paroi (114) est traversé par une ouverture (180) dans ladite région d'extrémité de l'évidement; un déplacement du moyen de broche l'amenant à faire saillie dans l'ouverture (180), dont le bord constitue ladite formation étagée.

9. Fermeture selon la revendication 8, dans laquelle la région d'extrémité du moyen de broche (160) présente une portion de diamètre relativement grande qui est forcée à travers l'ouverture (180) par ledit déplacement et qui s'engage ensuite en dessous de l'élément de paroi (160) pour restreindre le retrait de l'élément de broche (160) et pour verrouiller ainsi l'élément formant bouchon (38) à l'élément de paroi (114).

10. Récipient (12) présentant une embouchure et un ensemble de fermeture pour fermer l'embouchure, ledit ensemble étant réalisé selon l'une des revendications précédentes.
